# EUROPEAN PATENT SPECIFICATION

(11) **EP 2 465 413 B1**
(45) Date of publication and mention of the grant of the patent: **13.03.2019**
(21) Application number: 11009432.3
(22) Date of filing: 29.11.2011
(51) Int. Cl.: A61B 3/113, A61B 3/12, A61B 3/10

(54) **Ophthalmologic apparatus and control method therefor**
Ophthalmologische Vorrichtung und Steuerverfahren dafür
Appareil ophtalmologique et son procédé de contrôle

(30) Priority: 17.12.2010 JP 2010281447
(43) Date of publication of application: 20.06.2012
(73) Proprietor: Canon Kabushiki Kaisha, Tokyo (JP)
(72) Inventor: Makihira, Tomoyuki, Tokyo (JP)
(74) Representative: WESER & Kollegen

(56) References cited:
- EP-A1- 2 184 004
- US-A1- 2006 228 011
- KAWAI H ET AL: "EYE MOVEMENT ANALYSIS SYSTEM USING FUNDUS IMAGES", PATTERN RECOGNITION, ELSEVIER, GB, vol. 19, no. 1, 1 January 1986 (1986-01-01), pages 77-84, XP000716581, ISSN: 0031-3203, DOI: 10.1016/0031-3203(86)90036-1

## Description

### BACKGROUND OF THE INVENTION

### Field of the Invention

The present invention relates to an ophthalmologic apparatus and a control method for the ophthalmologic apparatus, and more particularly, to an ophthalmologic apparatus for measuring a movement of an eyeball and a control method for the ophthalmologic apparatus.

### Description of the Related Art

In recent years, increasing attention has been focused on apparatuses for measuring a movement of an eyeball. The reason for this attention is that if the movement of the eyeball can be measured, results thereof can be applied to visual field test, a fundus image photographing apparatus that requires a higher-resolution image, and the like, thereby enabling more accurate fundus examination.

There are various methods for measuring the movement of the eyeball, such as a corneal reflection method (Purkinje image) and a search coil method. Of those methods, various studies have been conducted on a method of measuring the movement of the eyeball based on a fundus image, which imposes less load on a subject.

In order to measure the movement of the eyeball with high accuracy by using a fundus image, the step of calculating a movement amount of a characteristic point needs to be processed at high speed after extracting the characteristic point from the fundus image, and searching for and detecting the characteristic point in an image to be processed. As the characteristic point of the fundus image, a macula, an optic disk, or the like is used. In the case of an affected eye or the like, the macula or the optic disk is frequently incomplete, and hence a blood vessel may be used as the characteristic point of the fundus image. Japanese Patent Application Laid-Open No. 2001-070247 discloses a method of extracting a characteristic point of a blood vessel.

The fundus image photographing apparatuses are categorized into fundus cameras that acquire, in one photographing operation, a fundus image covering an entire area, and scanning ophthalmoscopes that acquire a fundus image by scanning a beam. The scanning ophthalmoscopes are further categorized into scanning laser ophthalmoscopes (SLOs) that irradiate the fundus with a laser spot and scans the laser beam, and line-scanning laser ophthalmoscopes (hereinbelow, referred to as LSLOs) that irradiate the fundus with a line-shaped laser beam and scans the line laser beam. It is considered that, though it takes time to take an image, the scanning ophthalmoscope can provide higher image quality (higher resolution and higher brightness) compared to the fundus camera. With regard to the LSLO, Japanese Patent Application Laid-Open No. 2005-529669 discloses a detailed configuration. In general, the characteristic point needs to be detected in order to measure the movement of the eyeball, and hence the scanning ophthalmoscope capable of taking images successively with high image quality is used.

In order to detect the movement of the eyeball with high accuracy from the fundus image acquired by the fundus image photographing apparatus as disclosed in Japanese Patent Application Laid-Open No. 2005-529669, the movement amount of the eyeball between the acquired images can be calculated by using the method described in Japanese Patent Application Laid-Open No. 2001-070247, that is, by extracting the characteristic point of the fundus and making a comparison between the positions of the characteristic points in the acquired images.

US 2006/228011 A1 discloses another line scan imager used to determine the motion of a subject, wherein each line of image data is compared with a reference image and the location of a matching line in the reference image reveals the displacement of the subject.

However, the scanning-type fundus image photographing apparatus executes scanning, and thus there are problems that it takes time to take an image and that the measurement of the movement of the eyeball becomes difficult or less accurate with a lapse of time.

### SUMMARY OF THE INVENTION

The present invention has an object to, in a scanning-type ophthalmologic apparatus, measure a movement amount of an eyeball at high speed.

In order to achieve the above-mentioned object, according to the present invention, there is provided the ophthalmologic apparatus according to claim 1.

Further, according to the present invention, there is provided the control method for an ophthalmologic apparatus according to claim 14 and the recording medium according to claim 15. The other claims relate to further developments.

According to the present invention, the effect of measuring the movement amount of the eyeball at high speed can be obtained.

Further features of the present invention will become apparent from the following description of exemplary embodiments with reference to the attached drawings.

### BRIEF DESCRIPTION OF THE DRAWINGS

FIG. 1 is a schematic diagram of a fundus image photographing apparatus (SLO) according to a first embodiment of the present invention.
FIG. 2 is a schematic diagram of a control part of the apparatus according to the first embodiment of the present invention.
FIG. 3 is a flow chart according to the first embodiment of the present invention.
FIG. 4 is a flow chart (process A) according to the first embodiment and a second embodiment of the present invention.
FIG. 5 is a flow chart (process B) according to the first embodiment and the second embodiment of the present invention.
FIG. 6 is a flow chart (process C) according to the first embodiment and the second embodiment of the present invention.
FIGS. 7A, 7B, 7C, 7D, and 7E illustrate SLO images and an outline of a flow according to the first embodiment of the present invention.
FIG. 8 shows a criterion for determining an eyeball measurement scanning area according to the first embodiment of the present invention.
FIG. 9 shows eyeball measurement results according to the first embodiment of the present invention.
FIG. 10 is a schematic diagram of a control part of an apparatus according to the second embodiment of the present invention.
FIG. 11 is a schematic diagram of an apparatus configuration according to the second embodiment of the present invention.
FIG. 12 illustrates a display example according to the second embodiment of the present invention.
FIG. 13 is a flow chart according to the second embodiment of the present invention.
FIG. 14 is a flow chart (process D) according to the second embodiment of the present invention.
FIGS. 15A, 15B, 15C, 15D, and 15E illustrate SLO images and an outline of a flow according to the second embodiment of the present invention.
FIG. 16 shows a criterion for determining an eyeball measurement scanning area according to the second embodiment of the present invention.
FIG. 17 is a flow chart according to a third embodiment of the present invention.
FIGS. 18A, 18B, 18C, 18D, 18E, 18F, and 18G illustrate SLO images and an outline of a flow according to the third embodiment of the present invention.

### DESCRIPTION OF THE EMBODIMENTS

Embodiments for carrying out the present invention are described in detail with reference to the attached drawings.

### First embodiment

Hereinbelow, a first embodiment of the present invention is described.

In an example described in this embodiment, after a fundus image is acquired and a plurality of characteristic points (also referred to as "plurality of characteristic images) is extracted, a fundus area to be scanned is set, thereby enabling high speed measurement of a movement of an eyeball.

### (Overall configuration of apparatus)

A fundus image photographing apparatus of this embodiment includes a scanning laser ophthalmoscope (SLO) photographing part and a control part.

### <SLO photographing part>

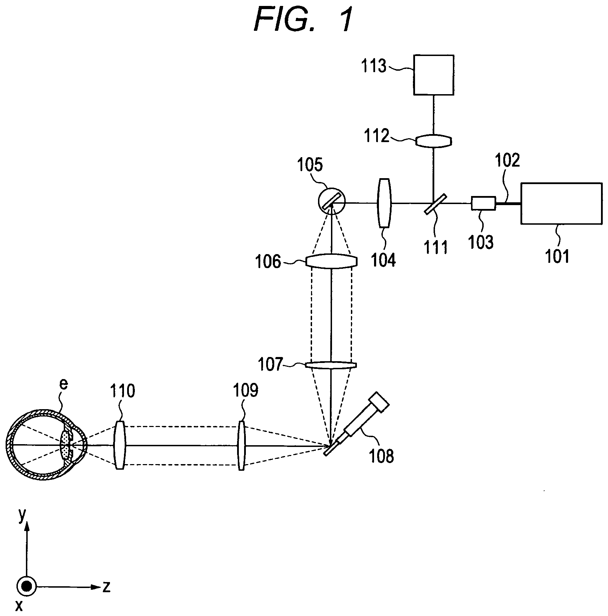

An optical configuration of the SLO photographing part is described with reference to FIG. 1.

As a laser light source 101, a semiconductor laser or a super luminescent diode (SLD) light source may be suitable for use. As for the wavelength to be used, in order to reduce glare for a subject and maintain the resolution at the time of fundus observation, a near-infrared wavelength region ranging from 700 nm to 1,000 nm is suitable for use. In this embodiment, a semiconductor laser having a wavelength of 780 nm is used.

A laser beam emitted from the laser light source 101 passes through a fiber 102, and is then emitted from a fiber collimator 103 as a collimated beam (measuring light). The emitted beam passes through a lens 104, an SLO scanner (Y) 105, and relay lenses 106 and 107, thereby being guided to an SLO scanner (X) 108. Further, the beam passes through a scan lens 109 and an ocular lens 110, and then enters an eye e to be inspected. As the SLO scanner (X) 108, a resonant scanner is used, and as the SLO scanner (Y) 105, a galvano scanner is used. Hereinafter, in the embodiments, coordinates are set with an eye axis direction, a horizontal direction with respect to a fundus image, and a vertical direction with respect to the fundus image corresponding to a z-direction, an x-direction, and a y-direction, respectively. In this embodiment, the x-direction is a main scanning direction, and the y-direction is a sub scanning direction.

The beam entering the eye e to be inspected irradiates, as a point beam, a fundus of the eye e to be inspected. This beam is reflected or scattered on the fundus of the eye e to be inspected, and then returns to a ring mirror 111 through the same optical path. Of the light backscattered after the beam enters the fundus, light (reflection light) that has passed through a pupil and its periphery is reflected by the ring mirror 111, and then passes through a lens 112 to be received by an avalanche photodiode (hereinbelow, referred to as APD) 113.

### <Control part>

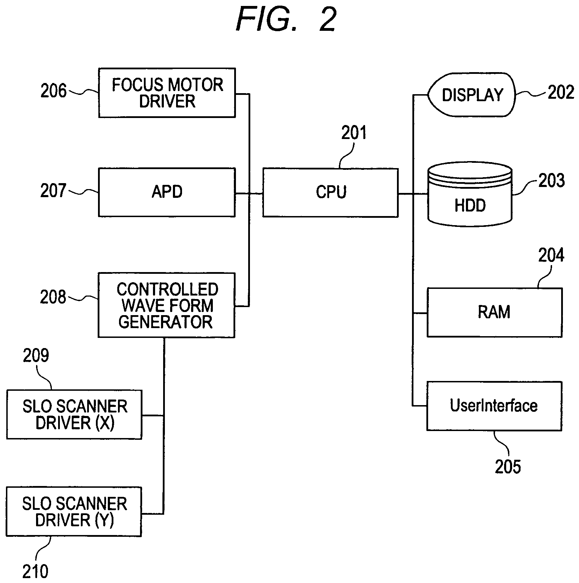

The control part of this embodiment is described with reference to FIG. 2.

A central processing unit (CPU) 201 is connected to a display device 202, a fixed disk drive 203, a main memory device (hereinbelow, referred to as memory) 204, a user interface 205, a focus motor driver 206, and a controlled wave form generator 208. The CPU 201 controls, via the controlled wave form generator 208 which generates the scanning wave form, an SLO scanner driver (X) 209 (driver for driving the SLO scanner (X) 108) and an SLO scanner driver (Y) 210 (driver for driving the SLO scanner (Y) 105). Further, an APD 207 (113) being a sensor of the SLO photographing part is connected to the CPU 201.

The CPU 201 uses the controlled wave form generator 208 to control the SLO scanner driver (X) 209 (108) and the SLO scanner driver (Y) 210 (105), thereby two-dimensionally scanning the fundus of the eye e to be inspected with the beam. The APD 207 (113) detects the reflection light generated after the scanning, thereby acquiring a two-dimensional image (SLO image) of the fundus.

According to the resolution or the number of pixels necessary for the SLO image, a readout frequency necessary in relation to scanning speed is set as an initial photographing condition. At the time of actual photographing, focus adjustment is executed with respect to the eye e to be inspected. In this case, the focus motor driver 206 executes the focus adjustment. A unit to be operated (moved) on this occasion is an ocular optical system, which is not illustrated in FIG. 1. For the execution of the focus adjustment, an examiner (operator) makes an input via the user interface 205 while checking the contrast of the SLO image displayed on the display device 202 of FIG. 2. After completion of the adjustment, the examiner gives a photographing instruction via the user interface 205.

### <Processes>

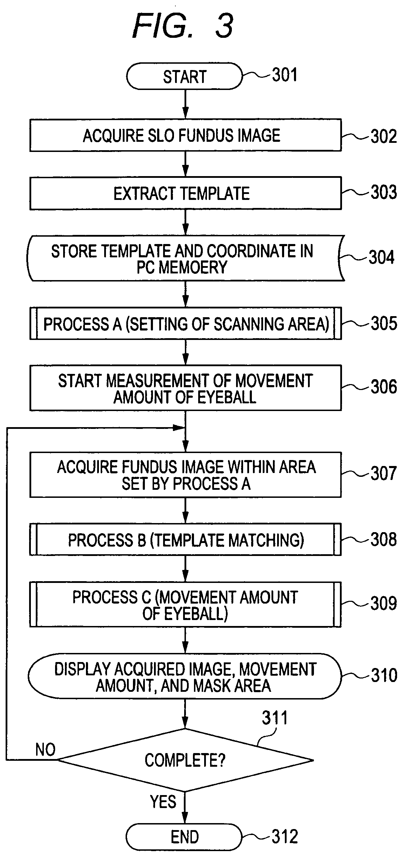

FIG. 3 is a flow chart illustrating processes of this embodiment. Note that, the following processes of the flow chart are implemented by the CPU 201 executing a program stored in advance in the memory. Note that, the CPU 201 may be construed as a computer.

First, the process is started in response to the photographing instruction given via the user interface 205 (Step 301), and, under control of the CPU 201, the SLO image is acquired under the initial photographing condition (Step 302: fundus image acquiring step). From the acquired SLO image, a characteristic point (in this embodiment, two-dimensional image: hereinbelow, referred to as template) of the image is extracted (Step 303: extracting step). A template identification number, the template (image), and the coordinates and size of the template, which are pieces of template information, are stored in the memory 204 (Step 304). Subsequently, in a process A, a scanning area of the beam on the fundus is set for measuring a movement of an eyeball (Step 305: setting step). The measurement of the movement of the eyeball is started (Step 306). The scanning area set in the process A is scanned with the beam to take an image of the scanning area, and the fundus image thus taken is acquired (Step 307: partial image acquiring step). In a process B, template matching is executed with respect to the taken fundus image (Step 308). In a process C, a comparison is made between the image coordinates of the template acquired in Step 303 and the image coordinates of the template acquired in Step 307, and, based on a result of the comparison, a movement amount of the eyeball is measured and calculated (Step 309: calculation step). The calculated movement amount of the fundus is displayed on a display being a display unit (Step 310), and after the measurement of the movement of the eyeball is completed or confirmed (Step 311), the process is ended when the measurement is completed (Step 312). When the measurement of the movement of the eyeball is not completed, the step of acquiring a partial image and the step of detecting a position corresponding to the prior characteristic point are executed repeatedly. Note that, the above-mentioned steps are executed, in the CPU 201, by parts that function as a fundus image acquiring unit, an extracting unit, a setting unit (determining unit), a partial image acquiring unit, a calculation unit, and a display control unit, respectively. Further, the detection step executed in Step 308 is executed by a part that functions as a detection unit for detecting the position of an area corresponding to the prior characteristic point in the partial image acquired as an image of the set scanning area. Further, after the above-mentioned steps or after the completion of the calculation step of Step 309, the SLO scanner driver (X) 209 and the SLO scanner driver (Y) 210 may be further operated based on the calculated movement amount. It is preferred that the control of those drivers be executed, in the CPU 201, by a part that functions as a control unit.

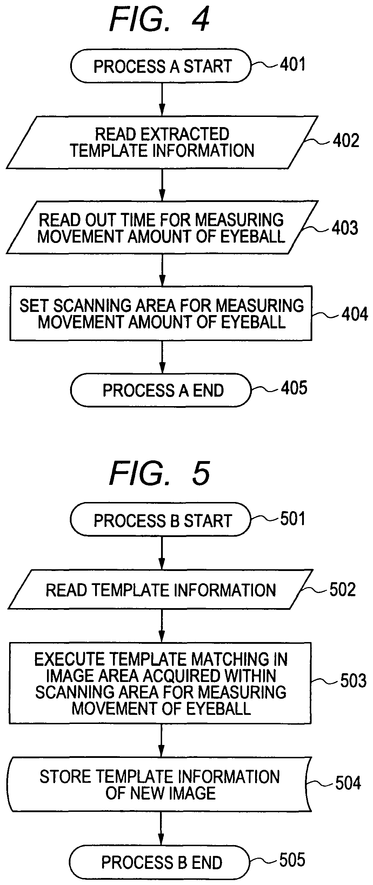

The process A (Step 305), which is part of the flow, is described with reference to FIG. 4. The size and coordinate information of the extracted template is read out from the memory 204 (Step 402). Based on the initial photographing condition, a time for measuring the movement of the eyeball is read out (Step 403). Based on the measurement time taken for the movement of the eyeball and the position (coordinates) of the template, the scanning area for measuring the movement of the eyeball is calculated and set (Step 404). Note that, in this embodiment, the scanning area to be set is obtained by narrowing the scanning area in the y-direction, that is, in the sub scanning direction of the measuring light, while the scanning area is left unchanged in the x-direction, with the result that the partial area for acquiring the partial image is obtained.

Subsequently, the process B (Step 305), which is part of the flow, is described with reference to FIG. 5. The template information is read out from the memory 204 on which the size and the coordinates of the template, and the measurement time of the movement of the eyeball are recorded (Step 502), and then, the template matching is executed within the newly-acquired fundus image (Step 503). The template matching of the present invention is executed based on at least one template (characteristic image) and the acquired partial image (also referred to as image of partial area or part of fundus image). Specifically, the template matching of the present invention refers to an operation of determining whether or not a characteristic image, a part having a brightness equal to or larger than a predetermined value, or the like within the acquired partial image corresponds to a part identical to the extracted or recorded template. Further, more specifically, the template matching of the present invention refers to an operation of searching the acquired partial image (part of the fundus image) for an image similar to the above-mentioned template. Note that, the actual operations for the determination and the search are processes that are commonly practiced in the template matching, and hence detailed description thereof is omitted. After the completion of the template matching, information on a matching image is stored in the memory 204 (Step 504). This process is executed for each acquired template.

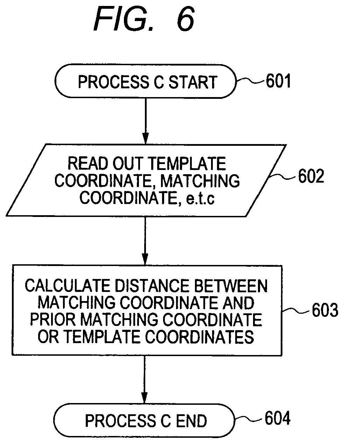

The process C (Step 309) is described with reference to FIG. 6. Matching coordinates of the prior process or template coordinates, and matching coordinates of the present process are read out (Step 602), and a coordinate difference for each template is calculated (Step 603), thereby calculating the movement amount of the eyeball in the fundus image based on the coordinate difference. The calculation of the movement amount is a commonly-practiced process, and hence detailed description thereof is omitted. Note that, in this example, first, the SLO image is acquired under the initial photographing condition, and then, the characteristic image is extracted from the SLO image, to thereby measure the movement of the eye to be inspected based on a relation with an image to be taken subsequently. However, for example, images of the fundus of the eye to be inspected may be taken successively at different photographing times, and, of those images, at least one fundus image may be selected and set as the image acquired in Step 302 in order to measure, based on that image, a subsequent movement of the eye to be inspected.

### (Movement measurement: specific example)

A specific example corresponding to the above-mentioned processes is described in the following.

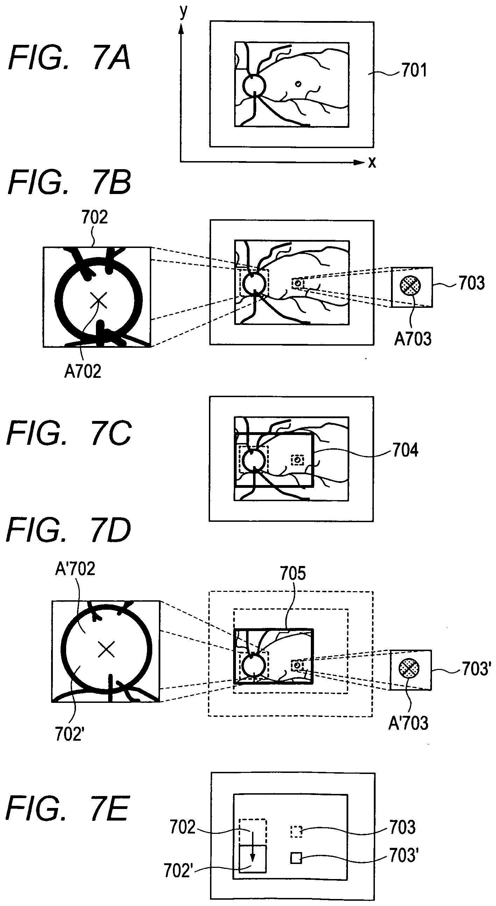

In the specific example, an SLO imaging apparatus being the above-mentioned fundus image photographing apparatus is used, and the eye to be inspected is measured for the movement of the eyeball for 20 seconds by an optical system capable of acquiring a fundus image corresponding to an area of 9 mm × 7 mm of the fundus. FIG. 7A illustrates the fundus image acquired under the initial photographing condition (represented by a schematic diagram, and hence scale is not accurate). A fundus image 701 is acquired through exposure of 0.05 seconds (corresponding to a frame rate of 20 Hz) by infrared light having a wavelength of 780 nm.

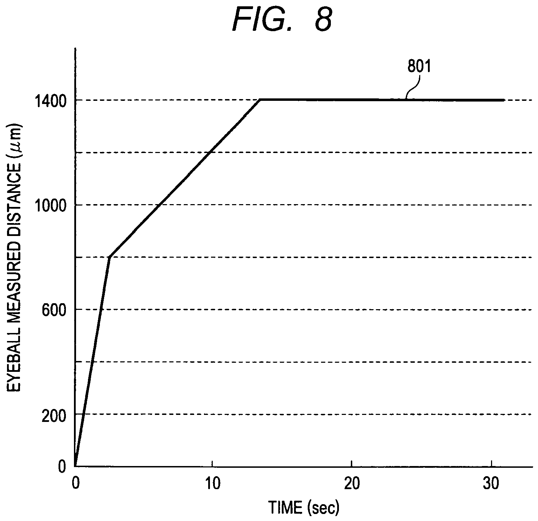

After the SLO image 701 is acquired, the templates are extracted at two points from the SLO image 701 (optic disk 702 and macula 703 of FIG. 7B). Templates 702 and 703 have areas of 1 mm × 1 mm and 0.5 mm × 0.5 mm, respectively, and center coordinates of the templates are represented by A702 (-2.5, 0) and A703 (0.5, 0), respectively. Those pieces of information are stored. The coordinates of the center of the SLO image 701 are represented by (0, 0), and the scanning area ranges from (-4.5, 3.5) to (4.5, -3.5). Based on the sizes and the coordinates of the images, the scanning area for the movement measurement is set. In this embodiment, as indicated by an image 704 of FIG. 7C, an area ranging from coordinates (-4.4, 1.9) to coordinates (2.15, -1.9) is set as the scanning area necessary for the measurement of the movement of the eyeball. However, in order to make the driving of the scanners less complicated, in this embodiment, the area is set to range from (-4.5, 1.9) to (4.5, -1.9). Here, FIG. 8 is a graph for describing a criterion for setting the scanning area, and shows a relation between a photographing time (measurement time of the movement of the eyeball) and a measurement area (eyeball measured distance), which are set based on the movement amount of the eyeball. This information is stored in the HDD 203 in advance, and the CPU 201 accesses the information so as to set the scanning area.

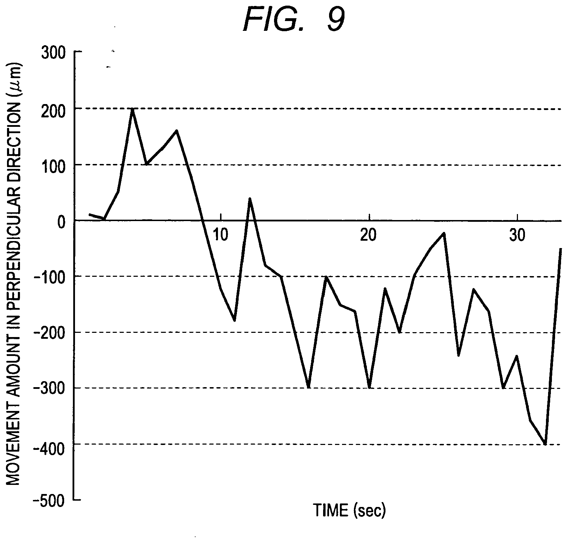

At the time of the measurement of the movement of the eyeball, the scanning area determined as in FIG. 7C is scanned, thereby acquiring a fundus image 705 as illustrated in FIG. 7D. The template matching process is executed to search for identical points in the respective images, thereby detecting areas 702' and 703'. Based on center coordinates A'702 (-2.5, -1.0) of the area 702' and center coordinates A'703 (0.5, -1.0) of the area 703', a moved distance (0, -1.0) of the eyeball is calculated (Fig.7E). The steps described above are repeatedly executed without changing the scanning area, and are then ended after the movement of the eyeball is measured for 20 seconds. Results of measuring the movement of the eyeball are displayed in real time on the display. Measurement results are shown in FIG. 9. A rate for acquiring data on the movement of the eyeball is 40 Hz.

In this manner, by setting an area that contains a plurality of characteristic points and scanning the area, that is, a partial area, the movement of the eyeball can be measured at high speed.

Further, at the time of setting the area, the size of the partial area is set according to the movement amount (or the moved distance) of the eyeball, which is predicted based on the photographing time of the fundus (or stored in advance), and hence it is possible to set such an area that is appropriate for the photographing condition.

Note that, in the embodiment described above, the number of characteristic images to be extracted is not particularly limited. In consideration of the subsequent operation of setting the partial area, it is preferred that a plurality of characteristic images be extracted. Further, with regard to the setting of the partial area, in order to enable the subsequent operation of the template matching, a partial area containing at least one characteristic image needs to be set.

### Second embodiment

Hereinbelow, a second embodiment of the present invention is described.

In an example described in this embodiment, after a fundus image is acquired to extract a characteristic point, an area to be scanned is set, thereby enabling high speed measurement of the movement of the eyeball. At the same time, by providing feedback to the optical coherence tomography (OCT) apparatus, an OCT image having high image quality (three-dimensional image having less positional displacement) is acquired.

### (Overall configuration of apparatus)

A fundus image photographing apparatus of this embodiment includes an OCT photographing part, an SLO photographing part, and a control part. Hereinbelow, the respective components are described in detail.

### <Optical configuration of OCT photographing part>

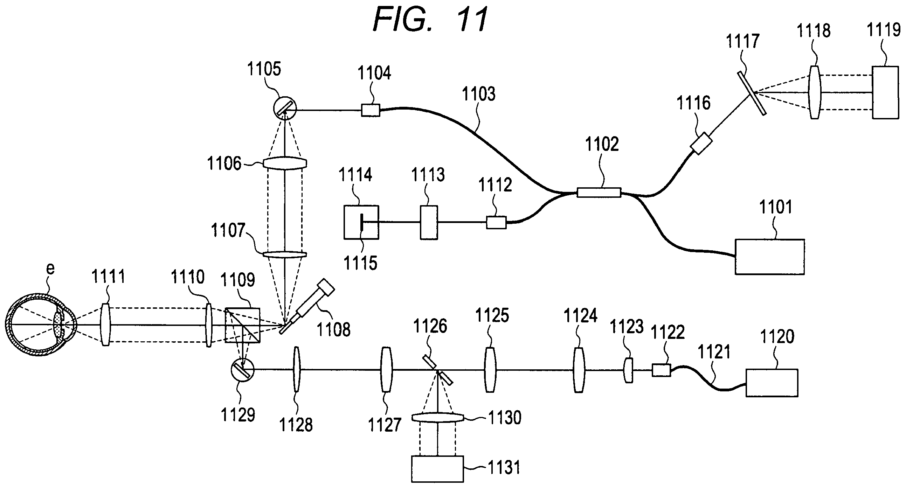

An optical configuration of the OCT photographing part of this embodiment is described with reference to FIG. 11.

As a low-coherence light source 1101, a super luminescent diode (SLD) light source or an amplified spontaneous emission (ASE) light source may be suitable for use. A swept-source (SS) light source may be used as well, but in this case, it is to be understood that the overall configuration needs to take a form of an SS-OCT system, which is different from the configuration illustrated in FIG. 11. As preferred wavelengths for the low-coherence light, the wavelengths in the vicinity of 850 nm and in the vicinity of 1,050 nm are suitable for use in fundus photographing. In this embodiment, an SLD light source having a center wavelength of 840 nm and a wavelength half-value width of 45 nm is used.

The low-coherence light emitted from the low-coherence light source 1101 passes through a fiber to enter a fiber coupler 1102, and is then split into measuring light (also referred to as OCT beam) and reference light. In this example, the configuration of an interferometer using fibers is illustrated, but a configuration in which a beam splitter is used in a spatial optical system may be used.

The measuring light passes through a fiber 1103, and is then emitted from a fiber collimator 1104 as collimated light. Further, the measuring light passes through an OCT scanner (Y) 1105, relay lenses 1106 and 1107, and further an OCT scanner (X) 1108, and is then transmitted through a dichroic beam splitter 1109 to pass through a scan lens 1110 and an ocular lens 1111, thereby entering an eye e to be inspected. In this example, as the OCT scanner (X) 1108 and the OCT scanner (Y) 1105, galvano scanners are used. The measuring light that has entered the eye e to be inspected is reflected on a retina, and then returns to the fiber coupler 1102 through the same optical path. The OCT scanner (Y) 1105 and the OCT scanner (X) 1108 serve as a scanning unit for scanning a fundus with the measuring light at the time of acquiring a tomographic image. Further, the OCT photographing part functions as a tomographic image acquiring unit for acquiring a tomographic image in the present invention.

The reference light is guided from the fiber coupler 1102 to a fiber collimator 1112, and is then emitted as collimated light. The emitted reference light passes through dispersion correction glass 1113, and is then reflected by a reference mirror 1115 provided to an optical path length varying stage 1114. The reference light reflected by the reference mirror 1115 returns to the fiber coupler 1102 through the same optical path.

The measuring light and the reference light, which have returned to the fiber coupler 1102, are combined by the fiber coupler 1102, and are then guided to a fiber collimator 1116. In this example, the combined light is referred to as interference light. The fiber collimator 1116, a grating 1117, a lens 1118, and a line sensor 1119 constitute a spectroscope. The interference light is converted to intensity information for each wavelength by the spectroscope, and then the intensity information is measured. In other words, the OCT photographing part of this embodiment employs a spectral domain system.

### <Optical configuration of SLO photographing part>

An optical configuration of the SLO photographing part for acquiring a fundus image is described with reference to the same figure, that is, FIG. 11. The SLO apparatus used in this embodiment is a line-scanning laser ophthalmoscope (LSLO), and, as a laser light source 1120, a semiconductor laser or an SLD light source may be suitable for use. As for the wavelength to be used, there is no limitation as long as the wavelength of the laser light source 1120 can be separated by the dichroic beam splitter 1109 from the wavelength of the low-coherence light source for the OCT. However, in consideration of the image quality of a fundus observation image, a near-infrared wavelength region ranging from 700 nm to 1,000 nm is suitable for use. In this embodiment, a wavelength of 760 nm is used. A laser beam emitted from the laser light source 1120 passes through a fiber 1121, and is then emitted from a fiber collimator 1122 as collimated light to enter a cylindrical lens 1123. In this embodiment, description is given by using the cylindrical lens, but there is no particular limitation as long as an optical element capable of generating a line beam is used. A Powell lens or a line beam shaper using a diffractive optical element may be used.

The beam (also referred to as measuring light or SLO beam) spread in the x-direction by the cylindrical lens 1123 is caused to pass through the center of a ring mirror 1126 by means of relay lenses 1124 and 1125, and then passes through relay lenses 1127 and 1128 to be guided to an SLO scanner (Y) 1129. As the SLO scanner (Y) 1129, a galvano scanner is used. Further, the beam is reflected by the dichroic beam splitter 1109, and then passes through the scan lens 1110 and the ocular lens 1111, thereby entering the eye e to be inspected. The dichroic beam splitter 1109 is configured to transmit the OCT beam and reflect the SLO beam. The beam that has entered the eye e to be inspected irradiates the fundus of the eye e to be inspected as a line-shaped beam. The line-shaped beam is reflected or scattered on the fundus of the eye e to be inspected, and then returns to the ring mirror 1126 through the same optical path.

The position of the ring mirror 1126 is in a conjugate relation with the position of the pupil of the eye e to be inspected. Of the light backscattered after the line beam enters the fundus, light (reflection light) that has passed through the pupil and its periphery is reflected by the ring mirror 1126, and then forms an image on a line sensor 1131 via a lens 1130. The intensity information detected by each element of the line sensor 1131 is transmitted to a computer (not shown), and is then subjected to processing to generate a fundus image.

The line beam is scanned in the vertical direction (y-direction) with respect to the fundus, thereby acquiring a two-dimensional fundus image.

### <Control part>

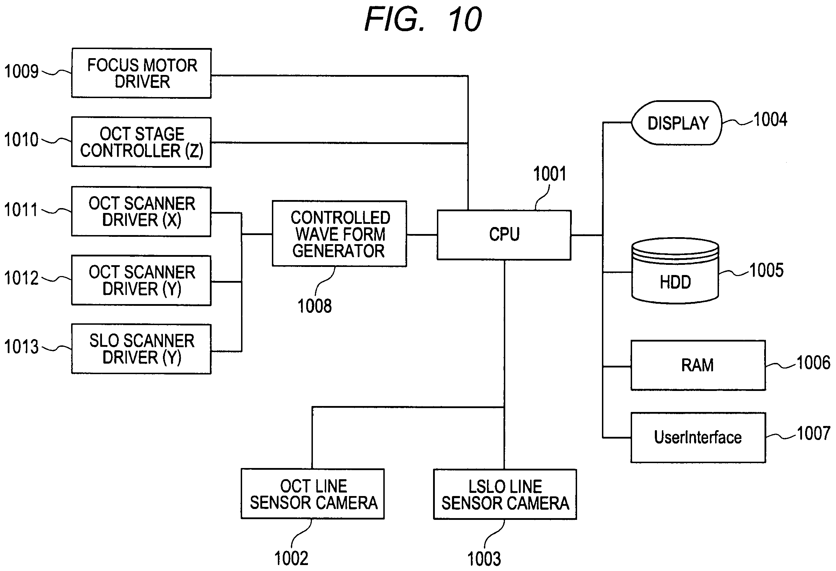

Next, the control part is described with reference to FIG. 10.

A central processing unit (CPU) 1001 is connected to a display device 1004, a fixed disk drive 1005, a main memory device 1006, and a user interface 1007. The CPU 1001 is also connected to a focus motor driver 1009 and an OCT stage controller 1010. Further, the CPU 1001 is connected to a controlled wave form generator 1008 which generates the scanning wave form, and controls, via the controlled wave form generator 1008, an OCT scanner driver (X) 1011 (driver for driving the OCT scanner (X) 1108), an OCT scanner driver (Y) 1012 (driver for driving the OCT scanner (Y) 1105), and an SLO scanner driver (Y) 1013 (driver for driving the SLO scanner (Y) 1129) . As a sensor of the spectroscope of the OCT photographing part, an OCT line sensor camera 1002 (1119) is connected, and as a sensor of the SLO photographing part, an LSLO line sensor camera 1003 (1131) is connected.

### <Process flow>

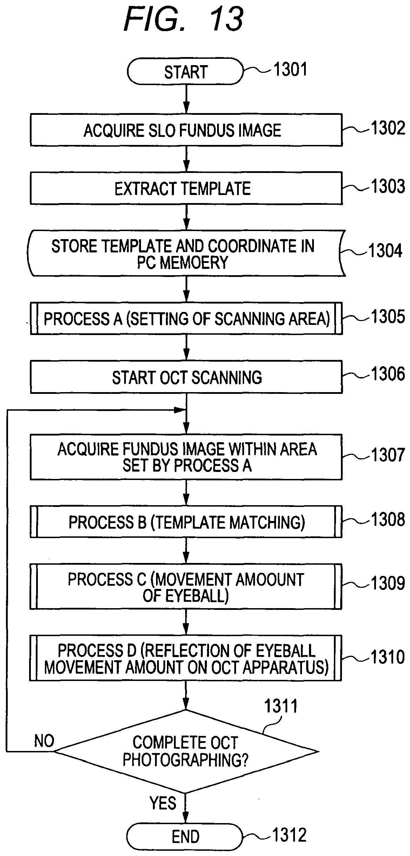

FIG. 13 illustrates an entire flow in which the movement of the eyeball is measured by using the above-mentioned apparatus, and an OCT image having high image quality is acquired by providing feedback to the scanner of the OCT apparatus. Note that, in the description given below, similar processes as those of the first embodiment are described briefly.

An SLO image is acquired (Step 1302), and a characteristic point (in this embodiment, referred to as template as well) is extracted from the SLO image (Step 1303). After the extraction of the template, the image, the coordinates, and the size, which are pieces of the template information, are stored (Step 1304). In this embodiment, a branching point of a blood vessel is used as the template.

Similarly to the case of the first embodiment, in a process A (scanning area setting), the scanning area is determined in consideration of the template extracting area described above and the OCT measurement time (Step 1305). The OCT scanning is started (Step 1306), and, at the same time, the area determined in the process A is scanned, to thereby acquire an SLO image (Step 1307). By using the acquired SLO images, the template matching is executed in a process B (Step 1308), and the movement of the eyeball is calculated in a process C (Step 1309). Further, in a process D, the scanner of the OCT apparatus is driven according to the calculated movement amount of the eyeball, and an OCT image at an appropriate position is acquired (Step 1310). The above-mentioned operation is executed repeatedly until the OCT photographing is completed (Step 1311).

The operations regarding the processes A, B, and C are similar to those of the first embodiment, and hence description thereof is omitted.

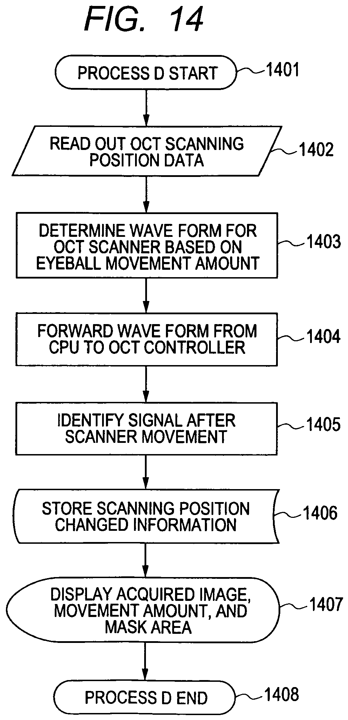

The process D (feedback to the OCT photographing part) is described with reference to FIG. 14. The CPU 1001 reads out scanning position data for the OCT photographing part from the controlled wave form generator 1008 (Step 1402), and causes the controlled wave form generator 1008 to generate, based on the movement amount of the eyeball determined from the SLO images, a wave form that takes into account the movement amount for the OCT scanner (Y) 1105 and the OCT scanner (X) 1108 (Step 1403). The generated wave form is forwarded to the OCT scanner driver (X) 1011 and the OCT scanner driver (Y) 1012 (Step 1404). Subsequently, after a signal for scanner movement sent from the controlled wave form generator 1008 is identified (Step 1405), scanning position changed information (information for correcting the scanning position based on the movement amount of the eyeball) is stored (Step 1406). The changed state, the OCT image, the SLO image (matching area and template position display), the remaining time, etc. are displayed (Step 1407).

### (Movement measurement: specific example)

A specific example corresponding to the processes described above is given in the following.

The SLO photographing part takes an image of the fundus in an area of 9 mm × 7 mm, and the OCT photographing part causes a camera to execute 70,000 A-scans per second. A B-scan image (fundus scanning area: 10 mm, the laser spot diameter: 20 µm) is constituted of 1,000 lines, and the measurement time is 4 seconds.

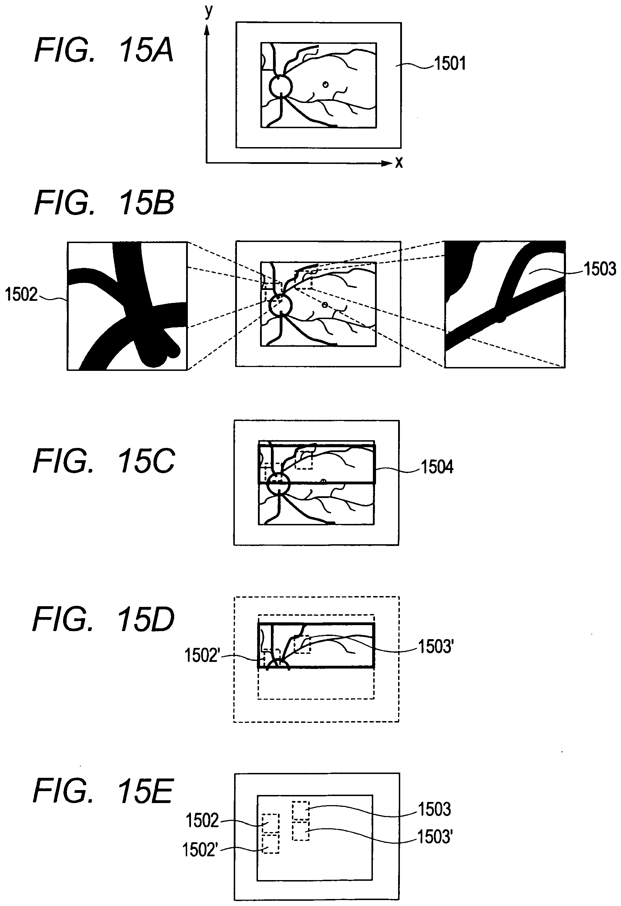

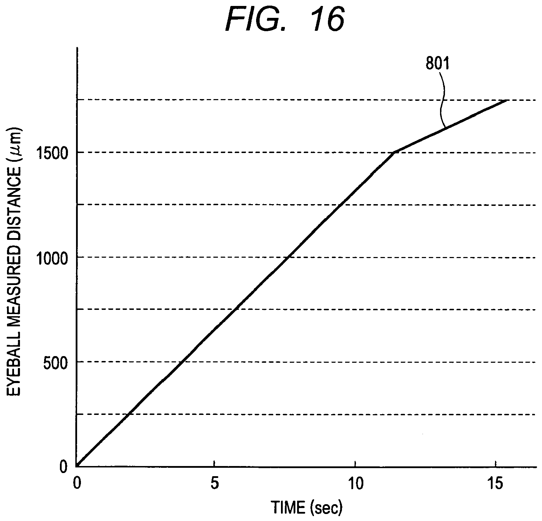

FIG. 15A illustrates an SLO image 1501 acquired by a line-scanning laser ophthalmoscope (LSLO). After the LSLO image is acquired, templates (1502 and 1503 of FIG. 15B) are extracted from the LSLO image. Information on the templates 1502 and 1503 is stored. In this embodiment, the coordinates of the templates 1502 and 1503 are (-3, 1) and (-2, 2), respectively. The center of the image 1501 is represented by (0, 0), and the coordinates of the templates represent vertices of the respective images. Subsequently, the scanning area is set. In this embodiment, the LSLO is used, and hence the length in the x-direction is constant, and the scanning area is determined in the y-direction. The measurement time is 4 seconds, and hence the eyeball measured distance is 0.5 mm as can be seen from FIG. 16. In consideration of the coordinates of the templates 1502 and 1503, the scanning area is set to range from +2.5 to 0.5 in y coordinate as is illustrated as an image 1504 of FIG. 15C. In this embodiment, the object to be inspected is an affected eye (no mydriatic), and hence the scanning area is determined based on the data of FIG. 16, which is different from that of the first embodiment. Note that, FIG. 16 is a graph for describing a criterion for setting the scanning area, and shows the relation between the photographing time and the measurement area (eyeball measured distance), which are set based on the movement amount of the eyeball. This information is stored in the HDD 1005 in advance, and the CPU 1001 accesses the information so as to set the scanning area. In other words, in this embodiment, the size of the partial area is set based on a time required for acquiring the tomographic image.

Subsequently, when the OCT photographing part starts to take an image of the fundus, the scanning of the LSLO is also executed at the same time. As illustrated in FIG. 15D, the area determined after the above-mentioned setting is set as the scanning area, and the fundus image is acquired by scanning the set area. After the image is acquired, the template matching is executed to detect areas 1502' and 1503', and the image information is stored. After that, a comparison is made between the information (coordinates) of the templates 1502 and 1503 and the information (coordinates) of the areas 1502' and 1503', to thereby measure the movement of the eyeball (Fig.15E). By reflecting the results of measuring the movement of the eyeball, a wave form for the OCT scanner (X) 1108 and the OCT scanner (Y) 1105 is generated, and the feedback is provided to the OCT scanner (Y) 1105 and the OCT scanner (X) 1108 of the OCT photographing part so that the OCT scanning is executed at a stable position. The operation described above is executed during the OCT photographing, and, as a result, an OCT image having high image quality (three-dimensional image having less positional displacement) can be acquired.

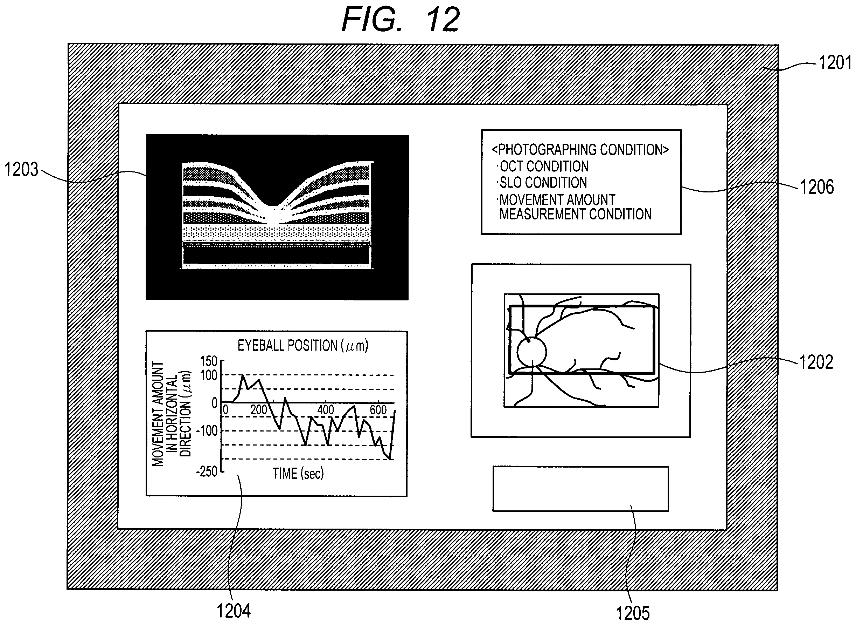

As illustrated in FIG. 12, the results obtained through the above-mentioned processes are reflected in real time to a display part 1201, to thereby display an SLO image 1202, an OCT image 1203, results 1204 of measuring the movement of the eyeball, a remaining measurement time 1205, photographing conditions 1206, etc. Accordingly, the user can check the operation.

As described above, by scanning a limited area containing a plurality of characteristic points, the movement of the eyeball can be measured at high speed, and also, by providing the feedback to the OCT apparatus, an OCT image having high image quality can be acquired.

### Third embodiment

Hereinbelow, a third embodiment of the present invention is described.

This embodiment relates to a mode regarding a fundus image photographing apparatus, which includes: a setting unit for acquiring a fundus image, extracting a plurality of characteristic images, and setting, in a photographing area, a partial area containing at least one characteristic image of the plurality of characteristic images; a partial image acquiring unit for acquiring an image of the partial area by scanning the set partial area with measuring light; and a measuring unit for measuring a movement of a fundus through template matching in which a similar point between the plurality of characteristic images and the image of the partial area is searched for and determined.

In an example described in this embodiment, after a plurality of characteristic points is extracted, an area to be scanned is set, thereby enabling high speed measurement of the movement of the eyeball. At the same time, by providing feedback to an OCT apparatus, an OCT image having high image quality (three-dimensional image having less positional displacement) is acquired.

### (Overall configuration of apparatus)

A fundus image photographing apparatus of this embodiment includes an OCT photographing part, an SLO photographing part, and a control part, and the apparatus configurations of the OCT photographing part, the SLO photographing part, and the control part are similar to those of the second embodiment, and hence description thereof is omitted.

### <Process flow>

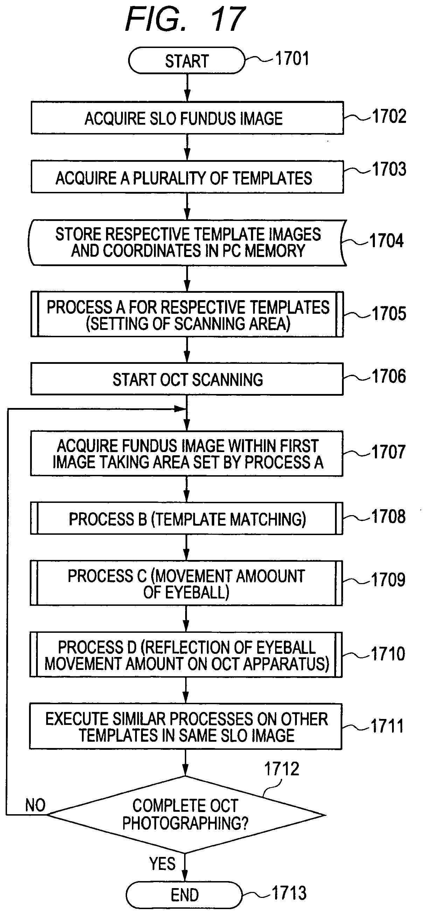

FIG. 17 illustrates an entire flow of this embodiment. Note that, in the description given below, similar processes as those of the first embodiment are described briefly.

An SLO image is acquired (Step 1702), and a plurality of characteristic points (in this embodiment, referred to as templates as well) is extracted from the SLO image (Step 1703). After the extraction of the templates, the image, the coordinates, and the size, which are pieces of the template information, are stored (Step 1704). In this embodiment, branching points of blood vessels are used as the templates.

Note that, it is preferred that the extraction of the plurality of characteristic points (characteristic images) be executed along the sub scanning direction at the time of scanning the fundus with the measuring light, that is, along the y-direction described above. In other words, it is preferred that the extraction be executed so that the characteristic points are arranged along the sub scanning direction. By executing the extraction operation in this mode, the setting of a partial area is executed more flexibly.

Similarly to the case of the first embodiment, in a process A (scanning area setting), the scanning area is determined in consideration of a first template extracting area described above and the OCT measurement time (Step 1705). The OCT scanning is started (Step 1706), and, at the same time, the area determined in the process A is scanned, to thereby acquire an SLO image (Step 1707). By using the acquired SLO images, the template matching is executed in a process B (Step 1708), and the movement of the eyeball is calculated in a process C (Step 1709). Further, in a process D, the scanner of the OCT apparatus is driven according to the calculated movement amount of the eyeball, and an OCT image at an appropriate position is acquired (Step 1710). The above-mentioned operation is executed sequentially for other templates (Step 1711). Further, a next SLO image is acquired, and the processes described above are repeated until the OCT photographing is completed (Step 1712).

The operations regarding the processes A, B, C, and D are similar to those of the second embodiment, and hence description thereof is omitted.

Note that, it is preferred that the determination of the scanning area in the process A, that is, the setting of the partial area according to the present invention, be executed along the sub scanning direction as in the case of the characteristic points. By setting the partial areas so as to be arranged along the sub scanning direction, for example, even when the number of extracted characteristic points is small, it becomes easier to include those characteristic points appropriately in the partial areas. Further, it is preferred that the partial areas be set sequentially in the sub scanning direction, and it is also preferred that the operation of the template matching between the SLO image acquired from the partial area and the characteristic point be executed for each partial area. With the above-mentioned mode, the process C can be executed with more accuracy.

### (Movement measurement: specific example)

A specific example corresponding to the processes described above is given in the following.

The SLO photographing part takes an image of the fundus in an area of 9 mm × 7 mm, and the OCT photographing part causes a camera to execute 70,000 A-scans per second. A B-scan image (fundus scanning area: 10 mm, laser spot diameter: 20 µm) is constituted of 1,000 lines, and the measurement time is 2 seconds.

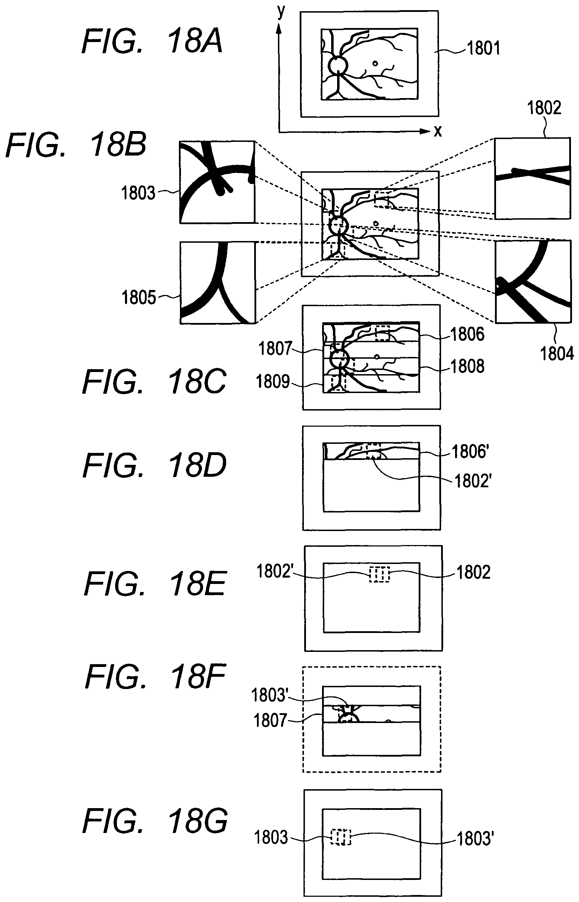

FIG. 18A illustrates an SLO image 1801 acquired by a line-scanning laser ophthalmoscope (LSLO). After the LSLO image is acquired, templates (1802, 1803, 1804, and 1805 of FIG. 18B) are extracted from the LSLO image. Information on the templates 1802 to 1805 is stored. In this embodiment, the coordinates of the templates 1802, 1802, 1803, 1804, and 1805 are (4, 5), (3, 1.5), (2, 2), and (1, 1.7), respectively. The lower left vertex of the image 1801 is represented by (0, 0), and the coordinates of the templates represent the center of the respective images. Subsequently, the scanning area is set. In this embodiment, the LSLO is used, and hence the length in the x-direction is constant, and the scanning area is determined in the y-direction. The measurement time is 2 seconds, and hence the eyeball measured distance is 0.25 mm as can be seen from FIG. 16. Thus, as the scanning areas, four areas 1806 to 1809 illustrated in FIG. 18C are set in order. Note that, the scanning areas may be set at random. In this embodiment, the object to be inspected is an affected eye (no mydriatic), and hence the scanning area is determined based on the data of FIG. 16 similarly to the case of the second embodiment.

Subsequently, when the OCT photographing part starts to take an image of the fundus, the scanning of the LSLO is also executed at the same time. As illustrated in FIG. 18D, one area is set as the scanning area in order from among the four areas 1806 to 1809 described above, and the fundus image is acquired by scanning the set area. After a new image 1806' corresponding to the set one area 1806 is acquired, the template matching is executed to detect an area 1802' for matching, and the image information is stored. After that, a comparison is made between the information (coordinates) of the template 1802 and the information (coordinates) of the area 1802', to thereby measure the movement of the eyeball (Fig.18E). By reflecting the results of measuring the movement of the eyeball, a wave form for the OCT scanner (X) 1108 and the OCT scanner (Y) 1105 is generated, and the feedback is provided to the OCT scanner (Y) 1105 and the OCT scanner (X) 1108 of the OCT photographing part so that the OCT scanning is executed at a stable position. Subsequently, as illustrated in FIG. 18F, a new fundus image 1807' corresponding to the next scanning area 1807 is acquired. Similarly to the case of the image 1806, the matching process is executed to detect an area 1803', and a comparison is made between the coordinates of the template 1803 and the coordinates of the area 1803', thereby calculating the movement amount of the eyeball (FIG.18G), which is then provided to the OCT apparatus as the feedback. Also with regard to the areas 1808 and 1809, similar processes are executed to provide the movement amount of the eyeball to the OCT apparatus as the feedback. After a new image corresponding to the area 1809 is acquired, the area corresponding to the initial area 1806 is scanned again. The scanning described above is executed during the OCT photographing, and, as a result, an OCT image having high image quality (three-dimensional image having less positional displacement) can be acquired.

As illustrated in FIG. 12, the results obtained through the above-mentioned processes are reflected in real time to the display part 1201, to thereby display the SLO image 1202, the OCT image 1203, the results 1204 of measuring the movement of the eyeball, the remaining measurement time 1205, the photographing conditions 1206, etc. Accordingly, the user can check the operation.

In this embodiment, four points are used, but similar processes can be executed as long as there are two or more points. Further, in this embodiment, the LSLO is used, but the SLO may be used as in the first embodiment.

As described above, by extracting a plurality of templates from the same SLO image and detecting the eyeball movement for each template of the same image, it is possible to detect the eyeball movement in a short period of time.

### Other embodiment

In the first and second embodiments, two characteristic points are used when the movement of the eyeball is measured, but any number of points may be used as long as there are two or more points. In order to determine the movement amount, the rotation, and the magnification change of the eyeball, it is preferred, if possible, that three or more points be used. Further, the characteristic point to be extracted is a small area having a characteristic, and thus the characteristic point may be a line segment or a two-dimensional image. Image correction is executed by using those characteristic points. In the embodiments, a crossing portion of a blood vessel, a branching portion of a blood vessel, an optic disk, and a macula are used as the characteristic point, but a surgical scar or the like may be used.

With the scanning-type fundus image photographing apparatus, a distortion occurs in a taken image due to involuntary movement of the eyeball. However, when the scanning area is small owing to the high speed scanning as in the first, second, and third embodiments, almost no distortion occurs.

The movement amount of the eyeball is calculated based on the amount of movement from the previous image, but instead may be calculated with the extracted template as a reference. Further, it is preferred that the criterion for the scanning area be changed depending on measurement conditions such as an internal fixation lamp, the state of a subject (such as affected eye or mydriasis), and the apparatus configuration.

In the second and third embodiments, the OCT photographing part is used as the apparatus to which the movement of the eyeball is reflected, but similar effects can be recognized for an ophthalmologic apparatus used for visual field test or the like. Further, the movement of the eyeball is corrected in real time for the ophthalmologic instrument, but the effects may also be provided by executing correction after the completion of the measurement or executing a post-process.

### (Other embodiment mode)

Further, the present invention is also implemented by executing the following process. Specifically, in this process, software (program) for implementing the functions of the above-mentioned embodiments is supplied to a system or an apparatus via a network or various kinds of storage medium, and a computer (or CPU, MPU, etc.) of the system or the apparatus reads out and executes the program.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An ophthalmologic apparatus for measuring a movement of an eye to be inspected, comprising:
a fundus image acquiring unit configured to acquire (step 302) a fundus image (701) of the eye; and
a measuring unit configured to measure (step 309) the movement of the eye,
**characterized by** further comprising:
an extracting unit configured to extract (step 303) at least one characteristic image (702) from the fundus image, said characteristic image being a two-dimensional part of the fundus image used as a template, showing a characteristic point of the fundus;
a determining unit configured to determine (step 305) a partial area (704) of the fundus image, including the at least one characteristic image and having a size set according to a predicted movement amount, based on a predetermined time of measuring the movement of the eye; and
a partial image acquiring unit configured to acquire (step 307) a partial fundus image (705) by scanning an area on the fundus of the eye corresponding to the partial area; and in that
the measuring unit is configured to measure the movement of the eye based on template matching of the at least one characteristic image, the template matching being executed with respect to the partial fundus image (step 308).

2. The ophthalmologic apparatus according to claim 1, wherein the characteristic point of the fundus includes a macula, an optic disk, a scar, a blood vessel, or a crossing or branching portion of a blood vessel.

3. The ophthalmologic apparatus according to claims 1 or 2, configured such that the determining unit determines the size of the partial area based on a time required for acquiring a tomographic image.

4. The ophthalmologic apparatus according to any one of claims 1 to 3, further comprising a display control unit configured to cause a display unit to display (step 310) the fundus image and the partial area superimposed on the fundus image.

5. The ophthalmologic apparatus according to any one of claims 1 to 4, further comprising a detection unit configured to detect (step 308) a position of an image (702') corresponding to the at least one characteristic image from the partial fundus image,
the apparatus configured such that the measuring unit determines, based on the detected position, a movement amount of the partial fundus image with respect to the fundus image, from which the at least one characteristic image has been extracted.

6. The ophthalmologic apparatus according to any one of claims 3 to 5, further comprising:
a tomographic image acquiring unit configured to acquire (step 1306) the tomographic image by using a scanning unit scanning the fundus with measuring light; and
a control unit configured to control (step 1310) the scanning unit based on the movement amount measured by the measuring unit.

7. The ophthalmologic apparatus according to claim 6,
configured such that the fundus image acquiring unit acquires the fundus image by scanning, with the measuring light, the fundus of the eye to be inspected, and
the partial image acquiring unit acquires the partial fundus image by scanning the area corresponding to the partial area with the measuring light.

8. The ophthalmologic apparatus according to claim 7,
configured such that the fundus image acquiring unit acquires the fundus image by scanning the measuring light in a main scanning direction and a sub scanning direction, and
the determining unit determines, as the partial area, a part of the fundus image narrowed in the sub scanning direction.

9. The ophthalmologic apparatus according to claim 7,
configured such that the fundus image acquiring unit acquires the fundus image (1801) by scanning the measuring light in a main scanning direction and a sub scanning direction,
the extracting unit extracts each of a plurality of characteristic images (1802-1805) along the sub scanning direction, and
the determining unit determines a plurality of the partial areas (1806-1809) each including one of the plurality of characteristic images.

10. The ophthalmologic apparatus according to claim 9, configured such that the measuring unit measures a movement of the fundus by executing template matching for each of the plurality of the partial areas determined in order in the sub scanning direction.

11. The ophthalmologic apparatus according to any one of claims 1 to 10, configured such that the template matching is executed so as to search the partial fundus image for an image similar to the at least one characteristic image.

12. The ophthalmologic apparatus according to claim 11, configured such that the measuring unit determines the movement amount of the fundus based on a position of the at least one characteristic image in the fundus image, and a position of the image similar to the at least one characteristic image in the partial fundus image.

13. The ophthalmologic apparatus according to any one of claims 1 to 12, configured such that the measurement of the movement of the eye to be inspected is executed by the measuring unit by searching the partial fundus image for the at least one characteristic image.

14. A control method for an ophthalmologic apparatus that measures a movement of an eye to be inspected, comprising:
acquiring (step 302) a fundus image (701) of the eye; and
measuring (step 309) the movement of the eye,
**characterized by** further comprising:
extracting (step 303) at least one characteristic image (702) from the fundus image, said characteristic image being a two-dimensional part of the fundus image used as a template, showing a characteristic point of the fundus;
determining (step 305) a partial area (704) of the fundus image, including the at least one characteristic image and having a size set according to a predicted movement amount, based on a predetermined time of measuring the movement of the eye; and
acquiring (step 307) a partial fundus image (705) by scanning an area on the fundus of the eye corresponding to the partial area; and in that
measuring the movement of the eye is based on template matching of the at least one characteristic image, the template matching being executed with respect to the partial fundus image (step 308).

15. A recording medium having recorded thereon a program for causing a computer to execute the steps of the control method for an ophthalmologic apparatus according to claim 14.

## Patentansprüche

1. Ophthalmologische Vorrichtung zum Messen einer Bewegung eines zu untersuchenden Auges, umfassend:
eine Augenhintergrundbild-Erfassungseinheit, die konfiguriert ist, ein Augenhintergrundbild (701) des Auges zu erfassen (Schritt 302); und
eine Messeinheit, die konfiguriert ist, die Bewegung des Auges zu messen (Schritt 309),
gekennzeichnet als des Weiteren umfassend:
eine Extrahiereinheit, die konfiguriert ist, mindestens ein charakteristisches Bild (702) aus dem Augenhintergrundbild zu extrahieren (Schritt 303), wobei das charakteristische Bild ein als Vorlage verwendeter, zweidimensionaler Teil des Augenhintergrundbildes ist, welches einen charakteristischen Punkt des Augenhintergrundes zeigt;
eine Bestimmungseinheit, die konfiguriert ist, ein Teilgebiet (704) des Augenhintergrundbildes zu bestimmen (Schritt 305), welches das mindestens eine charakteristische Bild enthält und eine gemäß einem vorhergesagten Bewegungsbetrag eingestellte Größe aufweist, und zwar basierend auf einer vorbestimmten Zeit des Messens der Augenbewegung; und
eine Teilbild-Erfassungseinheit, die konfiguriert ist, ein Augenhintergrundteilbild (705) durch Scannen eines dem Teilgebiet entsprechenden Gebiets auf dem Augenhintergrund zu erfassen (Schritt 307); und dadurch dass
die Messeinheit konfiguriert ist, die Bewegung des Auges basierend auf Vorlagenabgleich mit dem mindestens einen charakteristischen Bild zu messen, wobei der Vorlagenabgleich hinsichtlich des Augenhintergrundteilbildes ausgeführt wird (Schritt 308).

2. Ophthalmologische Vorrichtung nach Anspruch 1, wobei der charakteristische Punkt des Augenhintergrundes eine Makula, einen Sehnervenkopf, eine Narbe, ein Blutgefäß oder einen Kreuzungs- oder Verzweigungsabschnitt eines Blutgefäßes beinhaltet.

3. Ophthalmologische Vorrichtung nach Anspruch 1 oder 2, die derart konfiguriert ist, dass die Bestimmungseinheit die Größe des Teilgebiets basierend auf einer für die Erfassung eines Tomographiebildes benötigten Zeit bestimmt.

4. Ophthalmologische Vorrichtung nach einem der Ansprüche 1 bis 3, ferner umfassend eine Anzeigesteuereinheit, die konfiguriert ist, eine Anzeigeeinheit zu veranlassen, das Augenhintergrundbild und das dem Augenhintergrundbild überlagerte Teilgebiet anzuzeigen (Schritt 310).

5. Ophthalmologische Vorrichtung nach einem der Ansprüche 1 bis 4, ferner umfassend eine Detektionseinheit, die konfiguriert ist, eine dem mindestens einen charakteristischen Bild entsprechende Position eines Bildes (702') aus dem Augenhintergrundteilbild zu detektieren (Schritt 308),
wobei die Vorrichtung derart konfiguriert ist, dass die Messeinheit basierend auf der detektierten Position einen Bewegungsbetrag des Augenhintergrundteilbildes hinsichtlich des Augenhintergrundbildes detektiert, aus dem das mindestens eine charakteristische Bild extrahiert worden ist.

6. Ophthalmologische Vorrichtung nach einem der Ansprüche 3 bis 5, ferner umfassend:
eine Tomographiebild-Erfassungseinheit, die konfiguriert ist, das Tomographiebild unter Verwendung einer den Augenhintergrund mit Messlicht scannenden Scaneinheit zu erfassen (Schritt 1306); und
eine Steuereinheit, die konfiguriert ist, die Scaneinheit basierend auf dem von der Messeinheit gemessenen Bewegungsbetrag zu steuern (Schritt 1310).

7. Ophthalmologische Vorrichtung nach Anspruch 6,
derart konfiguriert, dass die Augenhintergrundbild-Erfassungseinheit das Augenhintergrundbild durch Scannen des Augenhintergrundes des zu untersuchenden Auges mit Messlicht erfasst, und
die Teilbild-Erfassungseinheit das Augenhintergrundteilbild durch Scannen des dem Teilgebiet entsprechenden Gebiets mit Messlicht erfasst.

8. Ophthalmologische Vorrichtung nach Anspruch 7,
derart konfiguriert, dass die Augenhintergrundbild-Erfassungseinheit das Augenhintergrundbild durch Scannen mit dem Messlicht in einer Hauptscanrichtung und einer Nebenscanrichtung erfasst, und
die Bestimmungseinheit einen in Nebenscanrichtung verengten Teil des Augenhintergrundbildes als das Teilgebiet bestimmt.

9. Ophthalmologische Vorrichtung nach Anspruch 7,
derart konfiguriert, dass die Augenhintergrundbild-Erfassungseinheit das Augenhintergrundbild (1801) durch Scannen mit dem Messlicht in einer Hauptscanrichtung und einer Nebenscanrichtung erfasst,
die Extrahiereinheit ein jeweiliges der mehreren charakteristischen Bilder (1802-1805) entlang der Nebenscanrichtung extrahiert, und
die Bestimmungseinheit mehrere der Teilgebiete (1806-1809) bestimmt, die jeweils eines der mehreren charakteristischen Bilder beinhalten.

10. Ophthalmologische Vorrichtung nach Anspruch 9, derart konfiguriert, dass die Messeinheit eine Bewegung des Augenhintergrundes durch Ausführen von Vorlagenabgleich für ein jeweiliges der mehreren Teilgebiete, die in Reihenfolge in der Nebenabtastrichtung bestimmt wurden, misst.

11. Ophthalmologische Vorrichtung nach einem der Ansprüche 1 bis 10, derart konfiguriert, dass der Vorlagenabgleich so ausgeführt wird, dass das Augenhintergrundteilbild nach einem dem mindestens einen charakteristischen Bild ähnlichen Bild durchsucht wird.

12. Ophthalmologische Vorrichtung nach Anspruch 11, derart konfiguriert, dass die Messeinheit den Bewegungsbetrag des Augenhintergrundes basierend auf einer Position des mindestens einen charakteristischen Bildes im Augenhintergrundbild bestimmt, sowie auf einer Position des dem mindestens einen charakteristischen Bild ähnlichen Bildes im Augenhintergrundteilbild.

13. Ophthalmologische Vorrichtung nach einem der Ansprüche 1 bis 12, derart konfiguriert, dass die Messung der Bewegung des zu untersuchenden Auges von der Messeinheit durch Durchsuchen des Augenhintergrundteilbildes nach dem mindestens einen charakteristischen Bild ausgeführt wird.

14. Steuerverfahren für eine ophthalmologische Vorrichtung, die eine Bewegung eines zu untersuchenden Auges misst, umfassend:
Erfassen (Schritt 302) eines Augenhintergrundbildes (701) des Auges; und
Messen (Schritt 309) der Bewegung des Auges,
gekennzeichnet als des Weiteren umfassend:
Extrahieren (Schritt 303) mindestens eines charakteristischen Bildes (702) aus dem Augenhintergrundbild, wobei das charakteristische Bild ein als Vorlage verwendeter, zweidimensionaler Teil des Augenhintergrundbildes ist, welches einen charakteristischen Punkt des Augenhintergrundes zeigt;
Bestimmen (Schritt 305) eines Teilgebietes (704) des Augenhintergrundbildes, welches das mindestens eine charakteristische Bild enthält und eine gemäß einem vorhergesagten Bewegungsbetrag eingestellte Größe aufweist, und zwar basierend auf einer vorbestimmten Zeit des Messens der Augenbewegung; und
Erfassen (Schritt 307) eines Augenhintergrundteilbildes (705) durch Scannen eines dem Teilgebiet entsprechenden Gebiets auf dem Augenhintergrund; und dadurch dass
das Messen der Bewegung des Auges auf Vorlagenabgleich mit dem mindestens einen charakteristischen Bild basiert, wobei der Vorlagenabgleich hinsichtlich des Augenhintergrundteilbildes ausgeführt wird (Schritt 308).

15. Aufzeichnungsmedium mit einem auf ihm gespeicherten Programm zum Veranlassen eines Computers, die Schritte des Steuerverfahrens für eine ophthalmologische Vorrichtung nach Anspruch 14 auszuführen.

## Revendications

1. Appareil ophtalmologique destiné à mesurer le mouvement d'un oeil à examiner, comprenant :
une unité d'acquisition d'image de fond d'oeil configurée pour acquérir (étape 302) une image de fond d'oeil (701) de l'oeil ; et
une unité de mesure configurée pour mesurer (étape 309) le mouvement de l'oeil, **caractérisé en ce qu'**il comprend en outre :
une unité d'extraction configurée pour extraire (étape 303) au moins une image caractéristique (702) de l'image de fond d'oeil, ladite image caractéristique étant une partie bidimensionnelle de l'image de fond d'oeil utilisée comme modèle, qui présente un point caractéristique du fond d'oeil ;
une unité de détermination configurée pour déterminer (étape 305) une zone partielle (704) de l'image de fond d'oeil, qui comprend ladite au moins une image caractéristique et qui présente une taille définie conformément à un degré de mouvement prévu, sur la base d'un temps prédéterminé pour mesurer le mouvement de l'oeil ; et
une unité d'acquisition d'image partielle configurée pour acquérir (étape 307) une image de fond d'oeil partielle (705) en balayant une zone sur le fond d'oeil de l'oeil correspondant à la zone partielle ; et **en ce que**
l'unité de mesure est configurée pour mesurer le mouvement de l'oeil sur la base d'une mise en correspondance de modèles de ladite au moins une image caractéristique, la mise en correspondance de modèles étant exécutée par rapport à l'image partielle du fond d'oeil (étape 308).

2. Appareil ophtalmologique selon la revendication 1, dans lequel le point caractéristique du fond d'oeil comprend une macula, un disque optique, une cicatrice, un vaisseau sanguin, ou une partie croisée ou ramifiée d'un vaisseau sanguin.

3. Appareil ophtalmologique selon les revendications 1 ou 2, configuré de façon que l'unité de détermination détermine la taille de la zone partielle sur la base d'un temps nécessaire pour acquérir une image tomographique.

4. Appareil ophtalmologique selon l'une quelconque des revendications 1 à 3, comprenant en outre une unité de commande d'affichage configurée pour amener une unité d'affichage à afficher (étape 310) l'image de fond d'oeil et la zone partielle superposée sur l'image de fond d'oeil.

5. Appareil ophtalmologique selon l'une quelconque des revendications 1 à 4, comprenant en outre une unité de détection configurée pour détecter (étape 308) une position d'une image (702') correspondant à ladite au moins une image caractéristique de l'image de fond d'oeil partielle,
l'appareil étant configuré de façon que l'unité de mesure détermine, sur la base de la position détectée, un degré de mouvement de l'image de fond d'oeil partielle par rapport à l'image de fond d'oeil à partir de laquelle ladite au moins une image caractéristique a été extraite.

6. Appareil ophtalmologique selon l'une quelconque des revendications 3 à 5, comprenant en outre :
une unité d'acquisition d'image tomographique configurée pour acquérir (étape 1306) l'image tomographique en utilisant une unité de balayage balayant le fond d'oeil à l'aide d'une lumière de mesure ; et
une unité de commande configurée pour commander (étape 1310) l'unité de balayage en fonction du degré de mouvement mesuré par l'unité de mesure.

7. Appareil ophtalmologique selon la revendication 6,
configuré de façon que l'unité d'acquisition d'image de fond d'oeil acquière l'image de fond d'oeil en balayant, à l'aide de la lumière de mesure, le fond d'oeil de l'oeil à examiner, et
l'unité d'acquisition d'image partielle acquiert l'image de fond d'oeil partielle en balayant la zone correspondant à la zone partielle à l'aide de la lumière de mesure.

8. Appareil ophtalmologique selon la revendication 7,
configuré de façon que l'unité d'acquisition d'image de fond d'oeil acquière l'image de fond d'oeil en animant la lumière de mesure d'un mouvement de balayage dans une direction de balayage principale et dans une direction de balayage secondaire, et
que l'unité de détermination détermine, en tant que surface partielle, une partie de l'image de fond d'oeil rétrécie dans la direction de balayage secondaire.

9. Appareil ophtalmologique selon la revendication 7,
configuré de façon que l'unité d'acquisition d'image de fond d'oeil acquière l'image de fond d'oeil (1801) en animant la lumière de mesure d'un mouvement de balayage dans une direction de balayage principale et dans une direction de balayage secondaire,
que l'unité d'extraction extraie chacune d'une pluralité d'images caractéristiques (1802-1805) le long de la direction de balayage secondaire, et
que l'unité de détermination détermine une pluralité des zones partielles (1806-1809) comprenant chacune l'une de la pluralité d'images caractéristiques.

10. Appareil ophtalmologique selon la revendication 9, configuré de façon que l'unité de mesure mesure un mouvement du fond d'oeil en exécutant une mise en correspondance de modèles pour chacune de la pluralité des zones partielles déterminées successivement dans la direction de balayage secondaire.

11. Appareil ophtalmologique selon l'une quelconque des revendications 1 à 10, configuré de façon que la mise en correspondance de modèles soit exécutée de manière à rechercher dans l'image de fond d'oeil partielle une image semblable à ladite au moins une image caractéristique.

12. Appareil ophtalmologique selon la revendication 11, configuré de façon que l'unité de mesure détermine le degré de mouvement du fond d'oeil sur la base d'une position de ladite au moins une image caractéristique dans l'image de fond d'oeil, et d'une position de l'image semblable à ladite au moins une image caractéristique dans l'image de fond d'oeil partielle.

13. Appareil ophtalmologique selon l'une quelconque des revendications 1 à 12, configuré de façon que la mesure du mouvement de l'oeil à examiner soit effectuée par l'unité de mesure en recherchant dans l'image de fond d'oeil partielle ladite au moins une image caractéristique.

14. Procédé de commande destiné à un appareil ophtalmologique qui mesure un mouvement d'un oeil à examiner, comprenant les étapes consistant à :
acquérir (étape 302) une image de fond d'oeil (701) de l'oeil ; et
mesurer (étape 309) le mouvement de l'oeil,
**caractérisé en ce qu'**il comprend en outre les étapes consistant à :
extraire (étape 303) au moins une image caractéristique (702) de l'image de fond d'oeil, ladite image caractéristique étant une partie bidimensionnelle de l'image de fond d'oeil utilisée comme modèle, qui présente un point caractéristique du fond d'oeil ;
déterminer (étape 305) une zone partielle (704) de l'image de fond d'oeil, qui comprend ladite au moins une image caractéristique et qui présente une taille définie conformément à une degré prévu de mouvement, sur la base d'un temps prédéterminé pour mesurer le mouvement de l'oeil ; et
acquérir (étape 307) une image de fond d'oeil partielle (705) en balayant une zone sur le fond d'oeil de l'oeil correspondant à la zone partielle ; et **en ce que**
la mesure du mouvement de l'oeil est basée sur une mise en correspondance de modèles de ladite au moins une image caractéristique, la mise en correspondance de modèles étant exécutée par rapport à l'image de fond d'oeil partielle (étape 308) .

15. Support d'enregistrement sur lequel est enregistré un programme pour amener un ordinateur à mettre en oeuvre les étapes du procédé de commande destiné à un appareil ophtalmologique selon la revendication 14.
